# EUROPÄISCHE PATENTSCHRIFT

(11) **EP 0 616 215 B1**
(45) Veröffentlichungstag und Bekanntmachung des Hinweises auf die Patenterteilung: **28.10.1998**
(21) Anmeldenummer: 94890042.8
(22) Anmeldetag: 16.02.1994
(51) Int. Cl.: G01N 33/38, G01N 1/02, B03B 9/06, B07B 13/08, G01N 9/00, B07C 5/36

(54) **Verfahren und Einrichtung zur Bestimmung der Reinheit von aufbereitetem Altglas**
Method and apparatus for the determination of the purity of prepared used glass
Méthode et appareil pour la détermination de la purété de déchets de verre

(30) Priorität: 15.03.1993 AT 506/93
(43) Veröffentlichungstag der Anmeldung: 21.09.1994
(73) Patentinhaber: Binder & Co. Aktiengesellschaft, 8200 Gleisdorf (AT)
(72) Erfinder: Gschweitl, Karlheinz, A-8211 Grospesendorf (AT)
(74) Vertreter: Kliment, Peter, Dipl.-Ing.

(56) Entgegenhaltungen:
- EP-A- 0 353 457
- EP-A- 0 461 616
- DE-C- 3 709 179
- US-A- 3 897 330
- US-A- 4 116 822
- AUFBEREITUNGS TECHNIK, Bd.33, Nr.8, August 1992, WIESBADEN DE Seiten 460 - 462, XP256701 W. THÜMMEL 'EINSATZ VON MOGENSEN VIBRO-STANGENSIZER BEIM ALTGLAS-RECYCLING'

## Beschreibung

Die Erfindung bezieht sich auf ein Verfahren zur Bestimmung der Reinheit von aufbereitetem Altglas gemäß dem Oberbegriff des Anspruches 1.

Bei der Aufbereitung von Altglas muß ein hohes Maß an Freiheit von Fremdstoffen gewährleistet sein. So darf das aufbereitete Altglas pro Tonne maximal 20g opaker Fremdkörper, wie Keramik, Porzellan oder Steine, und maximal 5g Nichteisen-Metalle enthalten. Diese hohen Reinheitsgrade sind erforderlich, da heute bereits bis zu 95% Altglas der Schmelze zugesetzt wird. Diese hohen Reinheitsgrade müssen von den Altglasaufbereitern garantiert und durch Proben des aufbereiteten Altglases nachgewiesen werden. Da diese Beprobungen in regelmäßigen Intervallen während der Aufbereitung stattfinden und aufgrund der sehr geringen zugelassenen Verunreinigungen, insbesondere durch Keramik, Steine, Porzellan und Nichteisenmetalle, große Probenmengen notwendig sind, gestaltet sich die Analyse des aufbereiteten Altglases sehr aufwendig. Dies ist auch durch den Umstand bedingt, daß die Proben heute manuell ausgewertet werden.

Bei der Beprobung wird bisher in der Regel die ÖNORM G 1032, Teil 1 bis 3, vom 5, 1992, die sich auf die Beprobung fester Brennstoffe bezieht, sinngemäß bei aufbereitetem Altglas angewandt. Dabei werden aus jeder Probenmenge die Fremdstoffe händisch sortiert, die noch enthaltenen Fremdstoffe aus Nichteisenmetallen und opaken Fremdstoffe, wie eben Keramik, Porzellan, Steine und dgl., entfernt und anschließend gewogen und in Relation zur Probenmenge gesetzt.

Ziel der Erfindung ist es, ein Verfahren anzugeben, das eine einfache Analyse einer Probenmenge aufbereiteten Altglases ermöglicht und die sich auch für eine automatisierte Durchführung eignet.

Erfindungsgemäß wird dies bei einem Verfahren der eingangs erwähnten Art durch die Merkmale des Kennzeichens des Anspruches 1 erreicht.

Ein Ausblasen von Fremdkörpern aus einem verunreinigten Strom von Glasteilchen im Bereich einer Freifallstrecke ist zwar an sich bei der Aufbereitung von Altglas, z.B. durch die EP 353 457 bekannt. Bei diesem bekannten Verfahren werden jedoch nur metallische Fremdkörper erfaßt und ausgeblasen.

Weiters ist aus der US 4 116 822 A ein Verfahren bekannt, bei dem der Altglasstrom durch einen Magnetabscheider geführt wird, der zur Abscheidung von ferromagnetischen Fremdkörpern dient. Anschließend werden mittels einer elektrostatisch aufladbaren Trommel, über die der verbleibende Altglasstrom geführt wird, Fremdkörper aus NE-Metallen, Steine, Porzellan u.dgl. abgeschieden, wobei jedoch keine Trennung nach der Art der Verunreinigung bzw. Fremdkörper und auch keine Wägung der einzelnen Fraktionen erfolgt.

Eine Untersuchung von Proben ist bei diesen bekannten Verfahren jedoch nicht vorgesehen. Für solche Untersuchungen stand bisher nur die sehr aufwendige manuelle Aussortierung und Wägung der einzelnen dabei erhaltenen Fraktionen zur Verfügung.

Durch die vorgeschlagenen Maßnahmen wird auf einfache Art eine Adaptierung eines bewährten Verfahrens für Analysezwecke sichergestellt.

Die Aussortierung von Fremdstoffen an sich aus einem Altglasstrom ist z.B. aus der EP 353 457 A bekannt, wobei nach diesem bekannten Verfahren der zu behandelnde Altglasstrom über eine Freifallstrecke rieseln gelassen wird, in deren Bereich Sensoren zur Erfassung von metallischen Fremdkörpern angeordnet sind, die ebenfalls im Bereich der Freifallstrecke angeordnete Blasdüsen steuern, die für das Ausblasen der Fremdkörper sorgen.

Durch die im Anspruch 2 angeführten Merkma!e wird vermindert, daß mehrere Partikel des aufbereiteten Altglases durch einen Feuchtigkeitsfilm aneinander haften und im Falle des Ausblasens eines dieser Teile gemeinsam ausgeblasen werden. So ergibt sich durch die im Anspruch 2 vorgeschlagenen Maßnahmen eine Erhöhung der Trennschärfe des Aussortierens von Fremdkörpern.

Durch die Merkmale des Anspruches 3 wird eine erhebliche Steigerung der Genauigkeit der Analyse mit relativ geringem Aufwand erreicht, da bei im wesentlichen gleichbleibendem Rohmaterial der Korrekturfaktor nur in größeren Zeitabständen, bzw. nach einer größeren Anzahl von Proben ermittelt werden muß und daher insgesamt, trotz einer hohen Analysegenauigkeit, nur wenig Handarbeit anfällt.

Durch die Merkmale des Anspruches 4 wird ein gezieltes Ausblasen der erfaßten Fremdkörper erleichtert.

Ein weiteres Ziel der Erfindung ist es, eine Einrichtung zur Durchführung des erfindungsgemäßen Verfahrens vorzuschlagen.

Durch die kennzeichnenden Merkmale des Anspruches 5 ergibt sich bei einer Einrichtung gemäß dem Oberbegriff des Anspruches 5 ein sehr einfacher Aufbau, bei dem durch die getrennten Reihen von Sensoren eine getrennte Erfassung von opaken Teilen und von Nichteisen-Teilen sichergestellt werden kann, die durch die in ebenfalls zwei Reihen angeordneten Ausblasdüsen getrennt aus der Probe des bereits aufbereiteten Glases ausgesondert werden können. Dadurch können die Antei!e an Verunreinigungen nach deren Art getrennt ermittelt werden.

Dabei ergibt sich durch die Merkmale des Anspruches 6 der Vorieil, daß die Auswertung weitgehend automatisiert werden kann.

Die Merkmale des Anspruches 7 sorgen für eine sichere Austreibung der Feuchtigkeit aus der Probe, wodurch ein Aneinanderhaften mehrerer Teilchen vermieden wird und die einzelnen Teilchen der Probe einzeln über die Freifallstrecke rieseln. Dadurch können die einzelnen Fremdkörper weitgehend allein ausgeblasen werden, ohne daß dabei auch eine nennenswerte Anzahl von Glasteilchen mit ausgeblasen wird.

Durch die Merkmale des Anspruches 8 wird eine unnötige Belastung der Umgebung von feinen Glasstäuben, wie sie sonst beim Ausblasen von Fremdkörpern zu erwarten wäre vermieden.

Die Erfindung wird nun anhand der Zeichnung näher erläutert. Dabei zeigen:

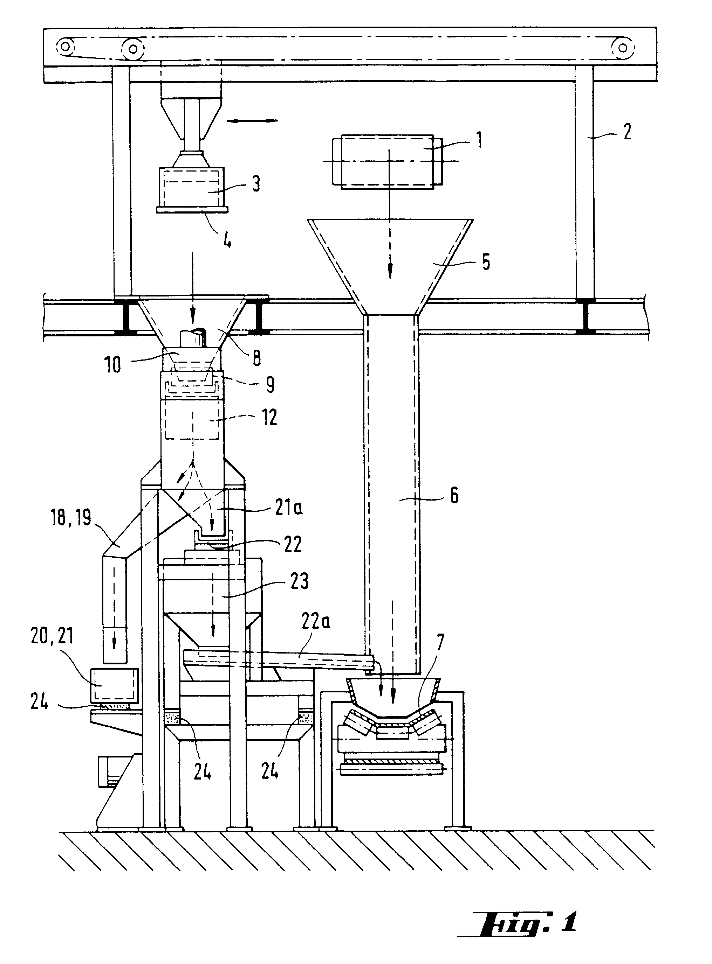

Fig. 1 eine Stirnansicht und

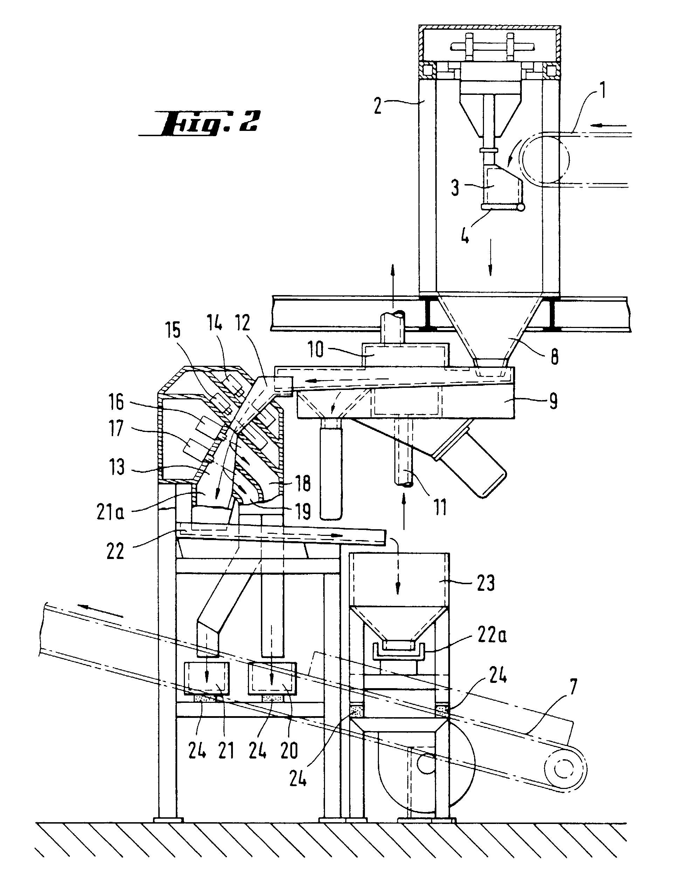

Fig. 2 eine Seitenansicht einer erfindungsgemäßen Einrichtung.

Im Endbereich eines das aufbereitete Altglas transportierenden Bandförderers 1 ist ein Gestell 2 angeordnet, das sich quer zur Förderrichtung des Bandförderers 1 erstreckt und in dem ein Probennehmer 3 quer zum Bandförderer 1 verfahrbar ist. Der Probennehmer 3 ist mit einem abklappbaren Boden 4 versehen, der ein Entleeren des Probennehmers 3 ermöglicht.

Im Bereich des Abwurfendes des Bandförderers 1 ist ein Trichter 5 vorgesehen, der mit einem Fallrohr 6 verbunden ist, das zu einem weiteren Förderer 7 führt, der das Altglas Einrichtungen zur Durchführung weiterer Produktionsschritte zuführt. Neben dem Trichter 5 ist ein weiterer Sammeltrichter 8 vorgesehen, der zur Aufnahme der Probenmenge dient, die vom Probennehmer 2 gezogen und aufgegeben werden.

Unterhalb des Trichters 8 ist ein Schwingförderer 9 angeordnet, an dessen Oberseite ein Anschluß 10 für eine Abluftleitung vorgesehen ist. Von der Unterseite her ist der Schwingförderer 9 über den Anschluß 11 mit Heißluft von z.B. 150°C beaufschlagbar, die zum Trocknen der Probenmenge dient.

An den Schwingförderer 9 schließt eine Rutsche 12 an, über die das durch den Schwingförderer einlagig geschichtete aufbereitete Altglas in eine Freifallstrecke 13 überführt wird.

Im Bereich der Rutsche 12 sind in zwei sich über die gesamte Breite der Rutsche 12 erstreckenden Reihen angeordnete Sensoren 14, 15 vorgesehen, von denen die Sensoren 14 Nichteisen-Metalle und die Sensoren 15 opake Fremdkörper, wie Keramik, Porzellan, Steine od. dgl. erfassen und Blasdüsen 16, 17 ansteuern, die mit Druckluft beaufschlagt sind und bei einer entsprechenden Ansteuerung durch die Sensoren kurz öffnen, um den Fremdkörper abzulenken und aus dem Glasstrom zu entfernen. Die Blasdüsen 16, 17 sind in zwei sich über die gesamte Breite der Freifallstrecke 13 erstreckenden Reihen angeordnet, wobei die Blasdüsen 16 von den Sensoren 14 und die Blasdüsen 17 von den Sensoren 15 gesteuert werden.

An der von den Blasdüsen 16, 17 abgekehrten Seite der Freifallstrecke sind den Blasdüsen 16, bzw. 17 zugeordnete Leiteinrichtungen 18, 19 angeordnet, die zu Auffangbehältern 20 für die Nichteisen-Metalle und 21 für opake Fremdkörper führen.

Das gereingte Glas der Probe wird über eine Auffangvorrichtung 21a und einen Schwingförderer 22 dem Reinglasbehälter 23 zugeführt und dort gesammelt.

Die Auffangbehälter 20, 21, sowie der Reinglasbehälter 23 stehen auf Wiegezellen 24, die mit einem Rechner verbunden sind, der den Anteil an Fremdkörper in der Probenmenge ermittelt.

Nach der Analyse der Probe wird der Reinglasbehälter 23 über eine Schwingrinne 22a zum Förderband 7 entleert und auf diese Weise der Rohstoff Altglas Einrichtungen zur Durchführung von weiteren Produktionsschritten zugeführt.

## Patentansprüche

1. Verfahren zur Bestimmung der Reinheit von aufbereitetem Altglas, bei dem die Probenmenge aus dem aufbereiteten Altglasstrom entnommen und die darin noch enthaltenen Fremdkörper, nach Nichteisen-Metallen und opaken Fremdstoffen, wie Keramik- und Porzellanteilchen und Steine getrennt, aussortiert, gewogen und in Relation zum Gewicht der gesamten Probe gesetzt werden, **dadurch gekennzeichnet,** daß die Probe in einer Lage geschichtet aufgeschüttet und über eine Freifallstrecke (13) rieseln gelassen wird und Fremdkörper aus Nichteisen-Metallen und opake Fremdkörper separat ausgeblasen und in separaten Behältern (20, 21) für Nichteisen-Metalle und opake Fremdstoffe aufgefangen werden.

2. Verfahren nach Anspruch 1, **dadurch gekennzeichnet,** daß die Probe vor dem Fall über die Freifallstrecke (13) getrocknet wird.

3. Verfahren nach Anspruch 1 oder 2, **dadurch gekennzeichnet,** daß die von mindestens einer Probe in den Behältern (20, 21) zur Aufnahme der Fremdkörper aus Nichteisen-Metallen bzw. der opaken Fremdkörper gesammelten Fremdkörper nach Glasteilen, die mit ausgeblasen wurden, handverlesen werden und aus dem Verhältnis von Glasteilen zu Fremdkörpern ein Korrekturfaktor errechnet wird, der bei der Ermittlung des Anteiles an den jeweiligen Fremdkörpern im aufbereiteten Altglas berücksichtigt wird.

4. Verfahren nach einem der Ansprüche 1 bis 3, **dadurch gekennzeichnet**, daß vor der Freifallstrecke (13) eine bestimmte Größe unterschreitende Teilchen abgesiebt werden und der Anteil der aussortierten Fremdkörper auf die verbleibende Probenmenge bezogen wird.

5. Einrichtung zur Durchführung des Verfahrens nach einem der Ansprüche 1 bis 4, mit einer Aufgabeeinrichtung (8), der ein Schwingförderer (9) und eine einer Rutsche (12) nachgeordnete Freifallstrecke (13) nachgeordnet ist, wobei Sensoren (14, 15) angeordnet sind, die in zwei in Fallrichtung voneinander distanzierten, quer zur Fallstrecke (13) verlaufenden Reihen angeordnete Blasdüsen (16, 17), die unterschiedlichen Auffangbehältern (20, 21) zugeordnet sind, steuern, und ein Reinglasbehälter (23) vorgesehen ist, **dadurch gekennzeichnet,** daß ein Probennehmer (3) vorgesehen ist, der in einen Strom von aufbereitetem Altglas einführbar und zur Aufgabeeinrichtung (8) verfahrbar ist, und die Sensoren (14, 15) im Bereich der Rutsche (12) der an diese anschließenden Freifallstrecke (13) in getrennten, quer zu diesen verlaufenden Reihen angeordnet und zur Erfassung von Nichteisen-Teilen und opaken Teilen vorgesehen sind.

6. Einrichtung nach Anspruch 5, **dadurch gekennzeichnet,** daß der Reinglasbehälter (23) und die Behälter (20, 21) mit Wägeeinrichtungen (24) verbunden sind.

7. Einrichtung nach Anspruch 5 oder 6, **dadurch gekennzeichnet,** daß der Schwingförderer (9) mit Heißluft beaufschlagbar ist.

8. Einrichtung nach einem der Ansprüche 5 bis 6, **dadurch gekennzeichnet,** daß der Rutsche (12) ein Sieb zum Abscheiden von ein bestimmtes Maß unterschreitenden Teilchen vorgeordnet ist.

## Claims

1. A method for determining the purity of processed waste glass, in which the sample quantity is taken from the processed waste glass stream and the foreign matter which is still contained therein and is separated according to non-ferrous metals and opaque foreign particles such as ceramic and porcelain particles and stones is sorted out, weighed and put in relation to the weight of the entire sample, characterized in that the sample is deposited in one stratified layer and is allowed to flow over a free-falling track (13) and foreign particles made of non-ferrous metals and opaque foreign particles are blown out separately and are collected in separate containers (20, 21) for non-ferrous metals and opaque foreign matter.

2. A method as claimed in claim 1, characterized in that the sample is dried before falling over the free-falling track (13).

3. A method as claimed in claim 1 or 2, characterized in that the foreign particles collected from at least one sample in the containers (20, 21) for receiving foreign particles from non-ferrous metals or opaque foreign particles are hand-picked with respect to glass parts which were also blown out and a correction factor is calculated from the ratio of glass parts to foreign particles which is considered in the determination of the share of the respective foreign particles in the processed waste glass.

4. A method as claimed in one of the claims 1 to 3, characterized in that before the free-falling track (13) particles are screened out which fall below a certain size and the share of the extracted foreign particles is put in relationship with with the remaining sample quantity.

5. A device to perform the method as claimed in one of the claims 1 to 4, with a feed device (8) which is provided downstream with an oscillating conveyor (9) and a free-falling track (13) which is arranged downstream of a chute (12), with sensors (14, 15) being arranged which control two blower nozzles (16, 17) which are assigned to different collecting containers (20, 21) and are arranged in two rows which are mutually distanced in the falling direction and extend transversally to the falling track (13), and with a pure-glass container (23) being provided, characterized in that a sampling device (3) is provided which is introduceable into a stream of processed waste glass and is movable towards the feed device (8), and the sensors (14, 15) are arranged in the zone of the chute (12) of the free-falling track (13), which is downstream of the same, in separate rows which extend transversally to the same and are provided for the detection of non-ferrous parts and opaque parts.

6. A device as claimed in claim 5, characterized in that the pure-glass container (23) and the containers (20, 21) are connected with weighing devices (24).

7. A device as claimed in claim 5 or 6, characterized in that the oscillating conveyor (9) can be charged with hot air.

8. A device as claimed in one of the claims 5 to 6, characterized in that the chute (12) is provided upstream with a screen for separating particles which fall below a certain size.

## Revendications

1. Procédé destiné à déterminer la pureté du verre récupéré traité, dans lequel les échantillons sont prélevés dans le flux de verre récupéré traité et les impuretés qui y sont encore présentes sont séparées en métaux non ferreux et corps étrangers opaques tels que les particules de céramique et porcelaine et les pierres, sont extraites des échantillons, sont pesées et comparées au poids de l'échantillon complet, **caractérisé en ce que** les échantillons sont répartis en une seule couche et s'écoulent dans un conduit à chute libre (13) et des impuretés constituées par des métaux non ferreux et des corps étrangers opaques sont éjectées de manière sélective et recueillies dans des récipients séparés (20, 21) pour métaux non ferreux et pour corps étrangers opaques.

2. Procédé selon la revendication 1, **caractérisé en ce que** l'échantillon est séché avant de pénétrer dans le conduit à chute libre (13).

3. Procédé selon la revendication 1 ou 2, **caractérisé en ce que** les impuretés d'au moins un échantillon recueillies dans les récipients (20, 21) destinés à recevoir respectivement les impuretés formées par les métaux non ferreux et les corps étrangers opaques, sont examinées à la main pour y déceler des particules de verre qui auraient été éjectées avec les impuretés et, à partir du rapport entre les particules de verre et les impuretés, on détermine un facteur de correction dont il est tenu compte lors de la détermination de la teneur en impuretés respectives dans le verre récupéré traité.

4. Procédé selon l'une quelconque des revendications 1 à 3, **caractérisé en ce qu**'en amont du conduit à chute libre (13) les particules de grosseur inférieure à une valeur définie sont évacuées par l'intermédiaire d'un tamis et le pourcentage d'impuretés évacuées par tri est comparé à la quantité restante de l'échantillon.

5. Dispositif destiné à la mise en oeuvre du procédé selon l'une quelconque des revendications 1 à 4, comprenant un dispositif de réception (8) et, en aval de celui-ci, un transporteur par secousses (9) et un conduit à chute libre (13) monté en aval d'une goulotte (12), des capteurs (14, 15), qui sont disposés en deux rangées distantes l'une de l'autre dans le sens de la chute et transversales au conduit à chute libre (13) et commandent des buses soufflantes (16, 17), qui sont attribuées à et commandent des récipients collecteurs séparés (20, 21), et un récipient collecteur de verre pur (23) étant prévu, caractérisé en ce qu'il est prévu un dispositif de prélèvement d'échantillons (3), qui peut être introduit dans un flux de verre récupéré traité et peut être déplacé vers le dispositif de réception (8), et les capteurs (14, 15), dans la zone de la goulotte (12) du conduit à chute libre (13) relié à celle-ci, sont disposés en rangées séparées, transversales à ces derniers et sont prévus pour déceler les particules non ferreuses et les particules opaques.

6. Dispositif selon la revendication 5, **caractérisé en ce que** le conteneur de verre pur (23) et les récipients collecteurs (20, 21) sont reliés à des dispositifs de pesage (24).

7. Dispositif selon la revendication 5 ou 6, **caractérisé en ce que** le transporteur par secousses (9) reçoit de l'air chaud.

8. Dispositif selon l'une quelconque des revendications 5 à 6, **caractérisé en ce qu**'un tamis, destiné à extraire des particules dont la grosseur est inférieure à une valeur définie, est monté en amont de la goulotte (12).
